# EUROPEAN PATENT SPECIFICATION

(11) **EP 2 516 682 B1**
(45) Date of publication and mention of the grant of the patent: **26.02.2020**
(21) Application number: 10839894.2
(22) Date of filing: 20.12.2010
(51) Int. Cl.: C12Q 3/00, C12M 1/00, C12M 1/02, C12M 1/34, C12M 1/36, C12M 3/06, G05B 13/00

(54) **A METHOD FOR CONTROLLING CULTURE PARAMETERS IN A BIOREACTOR**
VERFAHREN ZUR STEUERUNG VON KULTURPARAMETERN IN EINEM BIOREAKTOR
PROCÉDÉ POUR CONTRÔLER LES PARAMÈTRES DE CULTURE DANS UN BIORÉACTEUR

(30) Priority: 22.12.2009 SE 0951006
(43) Date of publication of application: 31.10.2012
(73) Proprietor: GE Healthcare Bio-Sciences AB, 75184 Uppsala (SE)
(72) Inventor: GEBAUER, Klaus, S-751 84 Uppsala (SE); LINDSKOG, Eva, S-751 84 Uppsala (SE); MAGNUSSON, Lars, S-751 84 Uppsala (SE)
(74) Representative: Cavill, Ross David
(86) International application number: PCT/SE2010/051425
(87) International publication number: WO 2011/078773

(56) References cited:
- WO-A1-2007/139742
- WO-A2-2007/134267
- WO-A2-2009/002772
- US-A- 6 029 101
- US-A- 6 029 101
- US-A1- 2005 112 542
- US-A1- 2005 112 542
- US-A1- 2005 272 146
- US-A1- 2005 272 146
- US-A1- 2006 019 388
- SVEN-OLOF ENFORS ET AL. BIOPROCESS TECHNOLOGY - FUNDAMENTALS AND APPLICATIONS, ROYAL INSTITUTE OF TECHNOLOGY January 2000, pages 28 - 30, XP008159854

## Description

### TECHNICAL FIELD OF THE INVENTION

The present invention relates to a method for controlling at least one culture parameter in a bioreactor bag provided in a bioreactor system. It further relates to a bioreactor system.

### BACKGROUND OF THE INVENTION

In bioreactors culture parameters such as temperature, pH or DO are controlled by different means. For example the temperature can be controlled by a heater blanket surrounding the bioreactor, a heating/cooling element under the bioreactor, a liquid containing jacket surrounding the bioreactor, by heating/cooling the environment that surrounds the bioreactor, by heating/cooling coils in the bioreactor, and by controlling the temperature of liquid or gas that is added to the bioreactor. pH can be controlled by adding acid or base, by adding gases, or by culture medium renewal. DO can be controlled by e.g. adding oxygen and/or nitrogen to the bioreactor headspace, by adding oxygen and/or nitrogen into the bulk fluid , by changing the stirrer speed, by changing the rocking rate, by changing the rocking angle and by changing the vertical/horizontal movement of the bioreactor.

Control of temperature and/or pH and/or DO can be performed by using feed-back loops consisting of a) a measuring device such as a sensor or an electrode immersed into or in close proximity to the culture, b) a software for control and c) a means for affecting the respective parameter. The measuring device records the process value of the respective parameter and the sensor signal provides indata for the control software. The software adjusts the output of the controller means dependent on the current process value and the process history, the controller type and a unique set of controller parameters. The parameters can be accessible in the graphical user interface or hidden in the software. Prior published material is US20050272146, WO2007139742 and WO2007134267. US20050272146 discloses a bioreactor system based on a disposable bioprocess bag, further including controllers, sensors and/or probes connected to a controller system. WO2007139742 discloses an automated cell culture system which incorporates disposable cultureware, further including a barcode reader and data gathering software used in an information gathering management system for controlled processing. WO2007134267 discloses a disposable material processing apparatus useable as a bioreactor or fermenter, including the use of a hollow mixing bag comprising a flexible film material, and means for control and a controller system.

### SUMMARY

An object of the invention is to provide an improved method for controlling culture parameters in a bioreactor.

This is achieved in a method according to claim 1 and in a bioreactor system according to claim 7.

Hereby culture parameters in bioreactor bags of for example different size, at different weight or containing cell cultures at different status can be controlled differently.

Suitable embodiments are described in the dependent claims.

### BRIEF DESCRIPTION OF THE DRAWINGS

Figure 1 is a schematic view of a bioreactor system according to one embodiment of the invention.
Figure 2 is a schematic view of a bioreactor system according to another embodiment of the invention.
Figure 3 is a flow chart of a method according to one embodiment of the invention.

### DETAILED DESCRIPTION

According to the invention a bioreactor system is provided comprising at least one bioreactor bag comprising a culture fluid. A control unit of the system is arranged to receive information about the at least one bioreactor and to control at least one culture parameter in dependence of said bioreactor information. The bioreactor information is the size of the bioreactor bag, and/or the weight of the bioreactor bag including the contents of the bag. Disclosed is that the bioreactor information could also be information about the cell culture status in the bioreactor bag. The cell culture status information could for example be retrieved from a sensor provided in the cell culture in the bioreactor bag and the information could be for example the growth phase of the cell culture, information about the metabolites or information about the biomass. The controlling of at least one culture parameter could include the changing of PID parameters or suitably choosing between different sets of PID parameters in dependence of said bioreactor information. Hereby the controlling of the culture parameter can be automatically adapted for different bioreactor features, above called bioreactor information. If two bioreactor bags are provided into the same bioreactor system the culture parameters of the bioreactor bags can be independently controlled in dependence of the respective bioreactor information. More details of specific examples are given below.

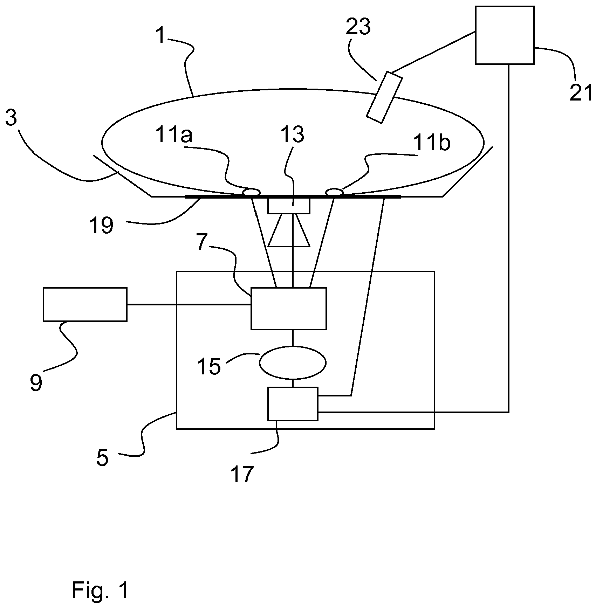

Figure 1 is a schematic view of a bioreactor system according to one example. In this example a bioreactor bag 1 is provided in a rocking tray 3 (also called a support). The rocking motion is suitable for providing proper gassing and mixing of the culture fluid inside the bag 1. However other gassing and mixing methods could also be used such as horizontal and/or vertical movement, stirring with impellers, gas additions into the bioreactor headspace and/or into the bulk fluid and/or pumps. The bioreactor system comprises furthermore a control unit 5. The control unit 5 comprises a receiving means 7 that in this embodiment is connected to an input means 9. Via the input means 9 a user is adapted to provide information to the control means 5 about the size of the bioreactor bag 1. This input from the user could either be a manual input or some kind of automatic reading such as bar code reading or RF reading of an RFID tag. The receiving means 7 is furthermore suitably connected to at least one sensor in the bioreactor bag 1. In this example the receiving means 7 is connected to two sensors 11a, 11b in the bioreactor bag 1. The sensors could be for example temperature sensors, pH sensors, DO sensors. The sensors 11a, 11b could also be sensors for measuring cell culture status, such as growth phase, biomass or metabolites. In this example the receiving means 7 is further connected to a load cell 13 provided on the tray 3 under the bioreactor bag 1. Hereby the weight of the bioreactor bag with its contents of culture fluid will be received as an input to the receiving means 7 in the control unit 5 when the bioreactor bag is placed on the tray.

The control unit 5 comprises further a determining means 15 connected to the receiving means 7. In this example the determining means 15 is adapted to use the information about the size of the bag, possibly together with information about at least one culture parameter received from the sensors 11a, 11b, to decide how to control this at least one culture parameter. In another embodiment information about the cell culture status from the sensors 11a, 11b is used to decide how to control the at least one culture parameter. In one embodiment of the invention there are predefined sets of parameters, possibly PID parameters, to use where each set is dedicated for each possible size (or in another embodiment: different possible cell culture states) of the bioreactor bag. In this case the determining means 15 only has to choose one of the sets of parameters depending of the size information received from the receiving means 7. In another example more complicated functions for determining an optimal control of a culture parameter could be provided. The functions could for example be dependent on both size and shape of the bag, the size of the tray, the weight of the liquid content within the bag, culture parameter values received from the sensors 11a, 11b and/or cell culture state information received from the sensors 11a, 11b. Furthermore, according to one example where a load cell 13 is provided, the determining means 15 could either use the size information or the weight of the bioreactor bag 1 for determining how to control the at least one culture parameter in the best way. A combination of the size information and the weight information could also be used.

The control unit 5 further comprises a controlling means 17 connected to the determining means 15 and adapted to control the at least one culture parameter according to what was determined in the determining means 15. In this example the controlling means 17 is connected to a heating and/or cooling means 19 provided under the tray in the form of a heater pad/cooler pad. Other possible heating and/or cooling means could be heating or cooling of the surrounding gases, heating or cooling of the inlet gas to the bioreactor and heating and/or cooling means inside the bioreactor. According to one example the heating and/or cooling means 19 will then control the temperature in the bioreactor bag 1 in dependence of the bioreactor information, which in this example is bag size but could also be cell culture status or weight as described above. In this example it is also shown that the controlling means 17 could be connected to some kind of adding means 21 connected to an inlet 23 to the bioreactor bag. This adding means 21 can be adapted to add for example one or more of oxygen, carbon dioxide, nitrogen, acid, base, specific nutrient concentrates or complete cell culture medium. Hereby other culture parameters than temperature can be controlled in dependence of the bioreactor information. These other culture parameters are for example pH, DO.

Since different bioreactor bag sizes can be used in the same bioreactor system this invention is very advantageous. When different bag sizes are used the culture parameters will change by different amounts when the same controlling will be applied. Heating a small bag size would for example be much faster than heating a large bag size. Hereby the adapted controlling according to the invention is advantageous and especially advantageous in bioreactor systems where the volume span is large.

A further advantage is that if two bioreactor bags are provided in the same tray the two bioreactor bags can be controlled independently and in dependence of each respective size or each respective other bioreactor information. Two bioreactor bags could be provided in the bioreactor system shown in Figure 1.

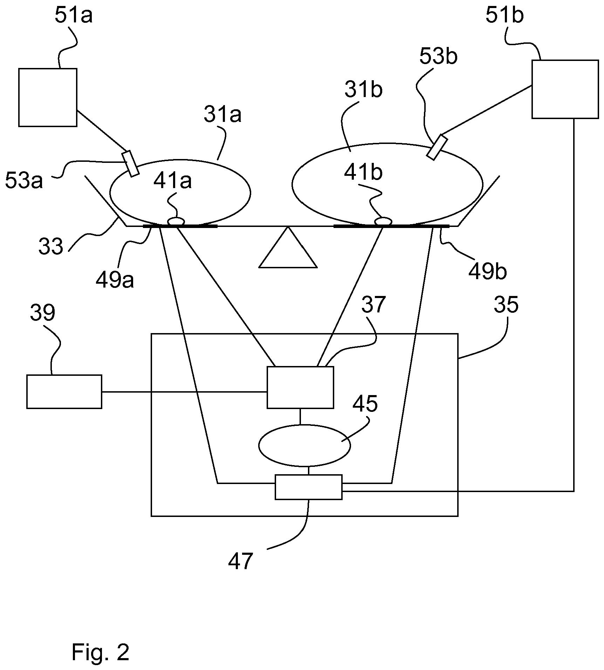

Figure 2 shows schematically a bioreactor system according to an example where two bioreactors 31a, 31b are provided in a rocking tray 33 (also called a support). A control unit 35 is provided in the bioreactor system. The control unit 35 comprises a receiving means 37 connected to an input means 39. Via the input means 39 a user is adapted to provide information to the control means 35 about the size of the two bioreactor bags 31a, 31b. This input from the user could either be a manual input or some kind of automatic reading such as bar code reading or RF reading of an RFID tag. The receiving means 37 is further suitably connected to at least one sensor 41a, 41b in each bioreactor bag 31a, 31b. The sensor could be for example a temperature sensor, pH sensor, DO sensor. The sensor could also possible measure cell culture status as defined above.

Similar to what was described in relation to Figure 1 the control unit 35 comprises further a determining means 45 connected to the receiving means 37 and adapted to use the bioreactor information for example about the sizes of the bags suitably together with information about at least one culture parameter received from the sensors 41a, 41b to decide how to control this at least one culture parameter independently in each of the two bioreactor bags 31a, 31b. As described before there could be provided different sets of parameters adapted for different bag sizes and then the determining means has to choose an appropriate set of parameters for each bag.

Furthermore, the control unit 35 also comprises a controlling means 47 connected to the determining means 45. The controlling means 47 is also connected to for example a heating and/or cooling means 49a, 49b connected to each bioreactor bag 31a, 31b and/or an adding means 51a, 51b connected one to each bioreactor bag 31a, 31b through an inlet 53a, 53b. The controlling is the same as described above. The advantage is as described above that these two bioreactor bags can be controlled independently and in dependence of each respective bag size (or other bioreactor information).

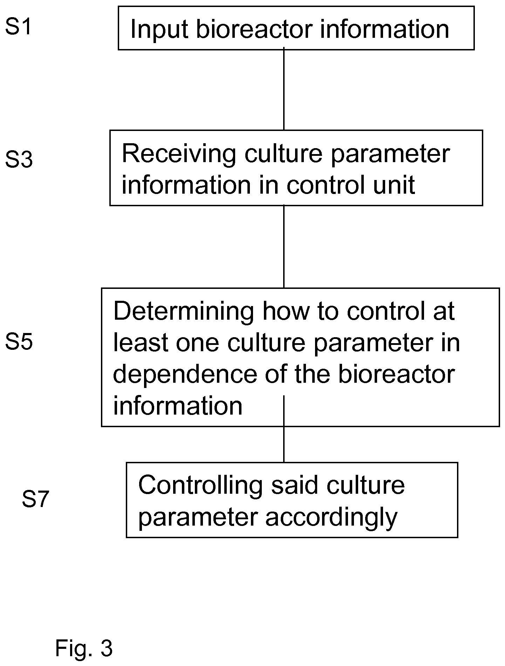

Figure 3 is a flow chart describing the method steps of one example of the invention.
S1: Bioreactor information of one or two bags 1, 31a, 31b provided on the tray 3, 33 is provided to the control unit 5, 35 of the bioreactor system, possibly via the input means 9, 39. In one sample the bioreactor information is size and then the providing of information could be either manual input of bag size or some kind of bar code reading of the bag size or RF reading of an RFID tag provided on the bioreactor bags. In other examples the providing of bioreactor information to the control unit could be from sensors in the bioreactor bag or from a load cell on the tray as described above.
S3: Receiving culture parameter information in the control unit from sensors 11a, 11b, 41a, 41b provided in the bioreactor bags 1, 31a, 31b.
S5: Determining how to control at least one culture parameter in each bioreactor bag in dependence of each respective bioreactor information.
S7: Controlling said culture parameter accordingly.

## Claims

1. A method for controlling at least one culture parameter in a bioreactor bag (1; 31a, 31b) provided in a bioreactor system, said method comprising the step of:
- providing bioreactor information to a control unit (5; 35) controlling the bioreactor system; the method being **characterised in that** it includes the further step of:
- controlling the at least one culture parameter in dependence of the bioreactor information;
wherein the bioreactor information is the size of the bioreactor bag, and/or the weight of the bioreactor bag; the method being further **characterised by** the step of :
- choosing one specific set among a number of different sets of control parameters in dependence of the bioreactor information.

2. A method according to claim 1, wherein the control parameters are PID control parameters.

3. A method according to any one of the preceding claims, wherein the culture parameter is temperature, and/or pH, and/or DO.

4. A method according to any one of the previous claims, wherein the bioreactor information is manually input to the control unit (5; 35) or read from a barcode or an RFID tag on the bioreactor bag.

5. A method according to any one of the previous claims, further comprising weighing the bioreactor bag (1) with culture fluid automatically when it is placed into the bioreactor system and using the weight of the bioreactor bag with culture fluid as bioreactor information for controlling the at least one culture parameter.

6. A method according to any one of the previous claims, wherein controlling the at least one culture parameter comprises heating and/or cooling and/or adding oxygen and/or adding carbon dioxide and/or adding nitrogen and/or adding acid and/or adding base and/or changing the gas flow to the bioreactor in dependence of the bioreactor information.

7. A bioreactor system comprising at least one bioreactor bag (1; 31a, 31b) comprising a culture fluid, the bioreactor system further comprising a control unit (5; 35) for controlling the bioreactor system, **characterised in that** said control unit (5; 35) comprises a receiving means (7; 37) adapted to receive bioreactor information and **in that** said control unit (5; 35) is adapted to control at least one culture parameter in the at least one bioreactor bag (1; 31a, 31b) in dependence of said bioreactor information, the system being **characterised in that** the bioreactor information is the size of the bioreactor bag, and/or the weight of the bioreactor bag; and **in that** the control unit chooses one specific set among a number of different sets of control parameters in dependence of the bioreactor information.

8. A bioreactor system according to claim 7, wherein the different sets of control parameters include PID parameters in dependence of the bioreactor information.

9. A bioreactor system according to claim 7 or 8, further comprising at least one sensor (11a, 11b, 41a, 41b) provided inside the bioreactor bag and connected to the control unit (5; 35), said sensor being adapted to measure the culture parameter to be controlled and/or at least one cell culture status such as biomass, cell growth phase or metabolites.

10. A bioreactor system according to any one of the claims 7, 8 or 9, wherein the control unit (5; 35) is adapted to control at least one of temperature, pH and DO in the at least one bioreactor bag in dependence of said bioreactor information.

11. A bioreactor system according to any one of the claims 7-10, further comprising at least one heating means and/or cooling means (19; 49a, 49b) adapted to heat or cool the at least one bioreactor bag (1; 31a, 31b), said heating and/or cooling means (19; 49a, 49b) being controlled by the control unit (5; 35) in dependence of said bioreactor information.

12. A bioreactor system according to any one of the claims 7-11, further comprising at least one adding means (21; 51a, 51b) adapted to add oxygen and/or carbon dioxide, and/or nitrogen and/or acid and/or base to the at least one bioreactor, said adding means (21; 51a, 51b) being controlled by the control unit (5; 35) in dependence of said bioreactor information.

13. A bioreactor system according to any one of the claims 7-12, wherein the receiving means (7; 37) is adapted to receive the bioreactor information by manual input to the control unit (5; 35) or by reading from a barcode or an RFID tag on the bioreactor bag.

14. A bioreactor system according to any one of the claims 7-13, further comprising at least one load cell (13) provided on a support (3) for the bioreactor bag and adapted to weigh the at least one bioreactor bag with culture fluid, said receiving means (7) further being connected to said load cell (13) and the control unit (5) is adapted to control at least one culture parameter in the at least one bioreactor bag (1) in dependence of the weight of the at least one bioreactor bag.

15. A bioreactor system according to any one of the claims 7-14, comprising two bioreactor bags (31a, 31b) on one support (33), wherein said control unit (35) being adapted to control each said bioreactor bag (31a, 31b) independently in dependence of the bioreactor information of each said bioreactor bag.

## Patentansprüche

1. Verfahren zum Steuern mindestens eines Kulturparameters in einem Bioreaktor-Beutel (1; 31a, 31b), der in einem Bioreaktorsystem bereitgestellt ist, wobei das Verfahren den folgenden Schritt umfasst:
- Bereitstellen von Bioreaktor-Informationen an eine Steuerungseinheit (5; 35), die das Bioreaktorsystem steuert; wobei das Verfahren **dadurch gekennzeichnet ist, dass** es weiter den folgenden Schritt beinhaltet:
- Steuern des mindestens einen Kulturparameters in Abhängigkeit von den Bioreaktor-Informationen;
wobei die Bioreaktor-Informationen die Größe des Bioreaktor-Beutels und/oder das Gewicht des Bioreaktor-Beutels sind; wobei das Verfahren weiter **gekennzeichnet ist durch** den folgenden Schritt:
- Wählen eines spezifischen Sets unter einer Anzahl von unterschiedlichen Sets von Steuerungsparametern in Abhängigkeit der Bioreaktor-Informationen.

2. Verfahren nach Anspruch 1, wobei die Steuerungsparameter PID-Steuerungsparameter sind.

3. Verfahren nach einem der vorstehenden Ansprüche, wobei der Kulturparameter Temperatur und/oder pH-Wert und/oder DO ist.

4. Verfahren nach einem der vorstehenden Ansprüche, wobei die Bioreaktor-Informationen manuell in die Steuerungseinheit (5; 35) eingegeben oder von einem Barcode oder einem RFID-Tag an dem Bioreaktor-Beutel ausgelesen werden.

5. Verfahren nach einem der vorstehenden Ansprüche, weiter umfassend das automatische Wiegen des Bioreaktor-Beutels (1) mit Kulturfluid, wenn er in das Bioreaktorsystem platziert wird und das Verwenden des Gewichts des Bioreaktor-Beutels mit Kulturfluid als Bioreaktor-Informationen zum Steuern des mindestens einen Kulturparameters.

6. Verfahren nach einem der vorstehenden Ansprüche, wobei das Steuern des mindestens einen Kulturparameters das Erwärmen und/oder Kühlen und/oder Hinzufügen von Sauerstoff und/oder Hinzufügen von Kohlendioxid und/oder Hinzufügen von Stickstoff und/oder Hinzufügen von Säure und/oder Hinzufügen von Base und/oder Ändern des Gasstroms zum Bioreaktor in Abhängigkeit der Bioreaktor-Informationen umfasst.

7. Bioreaktorsystem, umfassend mindestens einen Bioreaktor-Beutel (1; 31a, 31b), der ein Kulturfluid umfasst, wobei das Bioreaktorsystem weiter eine Steuerungseinheit (5; 35) zum Steuern des Bioreaktorsystems umfasst, **dadurch gekennzeichnet, dass** die Steuerungseinheit (5; 35) ein Empfangsmittel (7; 37) umfasst, angepasst, um Bioreaktor-Informationen zu empfangen, und dadurch, dass die Steuerungseinheit (5; 35) angepasst ist, um mindestens einen Kulturparameter in dem mindestens einen Bioreaktor-Beutel (1; 31a, 31b) in Abhängigkeit der Bioreaktor-Informationen zu steuern, wobei das System **dadurch gekennzeichnet ist, dass** die Bioreaktor-Informationen die Größe des Bioreaktor-Beutels und/oder das Gewicht des Bioreaktor-Beutels sind;
und dadurch, dass die Steuerungseinheit ein spezifisches Set unter einer Anzahl von unterschiedlichen Sets von Steuerungsparametern in Abhängigkeit der Bioreaktor-Informationen wählt.

8. Bioreaktorsystem nach Anspruch 7, wobei die unterschiedlichen Sets an Steuerungsparametern PID-Parameter in Abhängigkeit der Bioreaktor-Informationen beinhalten.

9. Bioreaktorsystem nach Anspruch 7 oder 8, weiter umfassend mindestens einen innerhalb des Bioreaktor-Beutels bereitgestellten und mit der Steuerungseinheit (5; 35) verbundenen Sensor (11a, 11b, 41a, 41b); wobei der Sensor angepasst ist, um den zu steuernden Kulturparameter und/oder mindestens einen Zellkulturstatus wie Biomasse, Zellwachstumsphase oder Metaboliten zu messen.

10. Bioreaktorsystem nach einem der Ansprüche 7, 8 oder 9, wobei die Steuerungseinheit (5; 35) angepasst ist, um mindestens eines von Temperatur, pH-Wert und DO in dem mindestens einen Bioreaktor-Beutel in Abhängigkeit der Bioreaktor-Informationen zu steuern.

11. Bioreaktorsystem nach einem der Ansprüche 7 bis 10, weiter umfassend mindestens ein Erwärmungsmittel und/oder Kühlungsmittel (19; 49a, 49b), angepasst, um den mindestens einen Bioreaktor-Beutel (1; 31a, 31b) zu erwärmen oder zu kühlen, wobei das Erwärmungs- und/oder Kühlungsmittel (19; 49a, 49b) durch die Steuerungseinheit (5; 35) in Abhängigkeit der Bioreaktor-Informationen gesteuert werden.

12. Bioreaktorsystem nach einem der Ansprüche 7 bis 11, weiter umfassend mindestens ein Hinzufügungsmittel (21; 51a, 51b), angepasst, um Sauerstoff und/oder Kohlendioxid und/oder Stickstoff und/oder Säure und/oder Base zu dem mindestens einen Bioreaktor hinzuzufügen, wobei das Hinzufügungsmittel (21; 51a, 51b) durch die Steuerungseinheit (5; 35) in Abhängigkeit der Bioreaktor-Informationen gesteuert wird.

13. Bioreaktorsystem nach einem der Ansprüche 7 bis 12, wobei das Empfangsmittel (7; 37) angepasst ist, um die Bioreaktor-Informationen durch manuelle Eingabe in die Steuerungseinheit (5; 35) oder durch Lesen von einem Barcode oder einem RFID-Tag an dem Bioreaktor-Beutel zu empfangen.

14. Bioreaktorsystem nach einem der Ansprüche 7 bis 13, weiter umfassend mindestens eine Wägezelle (13), bereitgestellt auf einem Träger (3) für den Bioreaktor-Beutel und angepasst, um den mindestens einen Bioreaktor-Beutel mit Kulturfluid zu wiegen, wobei das Empfangsmittel (7) weiter mit der Wägezelle (13) verbunden ist und die Steuerungseinheit (5) angepasst ist, um mindestens einen Kulturparameter in dem mindestens einen Bioreaktor-Beutel (1) in Abhängigkeit des Gewichts des mindestens einen Bioreaktor-Beutels zu steuern.

15. Bioreaktorsystem nach einem der Ansprüche 7 bis 14, umfassend zwei Bioreaktor-Beutel (31a, 31b) auf einem Träger (33), wobei die Steuerungseinheit (35) angepasst ist, um jeden Bioreaktor-Beutel (31a, 31b) unabhängig in Abhängigkeit der Bioreaktor-Informationen von jedem Bioreaktor-Beutel zu steuern.

## Revendications

1. Procédé de commande d'au moins un paramètre de culture dans un sac de bioréacteur (1; 31a, 31b) fourni dans un système de bioréacteur, ledit procédé comprenant l'étape consistant à :
- fournir des informations de bioréacteur à une unité de commande (5 ; 35) commandant le système de bioréacteur ; le procédé étant **caractérisé en ce qu'**il inclut l'étape supplémentaire consistant à :
- commander l'au moins un paramètre de culture vis-à-vis des informations de bioréacteur ;
dans lequel les informations de bioréacteur sont la taille du sac de bioréacteur, et/ou le poids du sac de bioréacteur ; le procédé étant en outre **caractérisé par** l'étape consistant à :
- choisir un ensemble spécifique parmi un nombre d'ensembles différents de paramètres de commande vis-à-vis des informations de bioréacteur.

2. Procédé selon la revendication 1, dans lequel les paramètres de commande sont des paramètres de commande PID.

3. Procédé selon l'une quelconque des revendications précédentes, dans lequel le paramètre de culture est la température, et/ou le pH, et/ou l'OD.

4. Procédé selon l'une quelconque des revendications précédentes, dans lequel les informations de bioréacteur sont entrées manuellement dans l'unité de commande (5 ; 35) ou lues depuis un code-barres ou une étiquette RFID sur le sac de bioréacteur.

5. Procédé selon l'une quelconque des revendications précédentes, comprenant en outre les étapes consistant à peser le sac de bioréacteur (1) avec du fluide de culture automatiquement lorsqu'il est placé dans le système de bioréacteur et utiliser le poids du sac de bioréacteur avec du fluide de culture comme informations de bioréacteur pour commander l'au moins un paramètre de culture.

6. Procédé selon l'une quelconque des revendications précédentes, dans lequel la commande de l'au moins un paramètre de culture comprend le chauffage et/ou le refroidissement et/ou l'ajout d'oxygène et/ou l'ajout de dioxyde de carbone et/ou l'ajout d'azote et/ou l'ajout d'acide et/ou l'ajout de base et/ou le changement du flux de gaz au bioréacteur vis-à-vis des informations de bioréacteur.

7. Système de bioréacteur comprenant au moins un sac de bioréacteur (1 ; 31a, 31b) comprenant un fluide de culture, le système de bioréacteur comprenant en outre une unité de commande (5 ; 35) pour commander le système de bioréacteur, **caractérisé en ce que** ladite unité de commande (5 ; 35) comprend un moyen de réception (7 ; 37) adapté pour recevoir des informations de bioréacteur et **en ce que** ladite unité de commande (5 ; 35) est adaptée pour commander au moins un paramètre de culture dans l'au moins un sac de bioréacteur (1 ; 31a, 31b) vis-à-vis desdites informations de bioréacteur, le système étant **caractérisé en ce que** les informations de bioréacteur sont la taille du sac de bioréacteur, et/ou le poids du sac de bioréacteur ;
et **en ce que** l'unité de commande choisit un ensemble spécifique parmi un nombre d'ensembles différents de paramètres de commande vis-à-vis des informations de bioréacteur.

8. Système de bioréacteur selon la revendication 7, dans lequel les ensembles différents de paramètres de commande incluent des paramètres PID vis-à-vis des informations de bioréacteur.

9. Système de bioréacteur selon la revendication 7 ou 8, comprenant en outre au moins un capteur (11a, 11b, 41a, 41b) fourni à l'intérieur du sac de bioréacteur et raccordé à l'unité de commande (5 ; 35), ledit capteur étant adapté pour mesurer le paramètre de culture à commander et/ou au moins un statut de culture cellulaire comme la biomasse, la phase de croissance cellulaire ou les métabolites.

10. Système de bioréacteur selon l'une quelconque des revendications 7, 8 ou 9, dans lequel l'unité de commande (5 ; 35) est adaptée pour commander au moins un parmi la température, le pH et l'OD dans l'au moins un sac de bioréacteur vis-à-vis desdites informations de bioréacteur.

11. Système de bioréacteur selon l'une quelconque des revendications 7 à 10, comprenant en outre au moins un moyen de chauffage et/ou un moyen de refroidissement (19 ; 49a, 49b) adapté pour chauffer ou refroidir l'au moins un sac de bioréacteur (1 ; 31a, 31b), ledit moyen de chauffage et/ou de refroidissement (19 ; 49a, 49b) étant commandé par l'unité de commande (5 ; 35) vis-à-vis desdites informations de bioréacteur.

12. Système de bioréacteur selon l'une quelconque des revendications 7 à 11, comprenant en outre au moins un moyen d'ajout (21 ; 51a, 51b) adapté pour ajouter de l'oxygène et/ou du dioxyde de carbone et/ou de l'azote et/ou de l'acide et/ou de la base à l'au moins un bioréacteur, ledit moyen d'ajout (21 ; 51a, 51b) étant commandé par l'unité de commande (5 ; 35) vis-à-vis desdites informations de bioréacteur.

13. Système de bioréacteur selon l'une quelconque des revendications 7 à 12, dans lequel le moyen de réception (7 ; 37) est adapté pour recevoir les informations de bioréacteur par entrée manuelle dans l'unité de commande (5 ; 35) ou en lisant depuis un code-barres ou une étiquette RFID sur le sac de bioréacteur.

14. Système de bioréacteur selon l'une quelconque des revendications 7 à 13, comprenant en outre au moins une cellule de charge (13) fournie sur un support (3) pour le sac de bioréacteur et adapté pour peser l'au moins un sac de bioréacteur avec du fluide de culture, ledit moyen de réception (7) étant en outre raccordé à ladite cellule de charge (13) et l'unité de commande (5) est adaptée pour commander au moins un paramètre de culture dans l'au moins un sac de bioréacteur (1) vis-à-vis du poids de l'au moins un sac de bioréacteur.

15. Système de bioréacteur selon l'une quelconque des revendications 7 à 14, comprenant deux sacs de bioréacteur (31a, 31b) sur un support (33), dans lequel ladite unité de commande (35) étant adaptée pour commander chaque dit sac de bioréacteur (31a, 31b) indépendamment vis-à-vis des informations de bioréacteur de chaque dit sac de bioréacteur.
